(19) Europäisches Patentamt
European Patent Office
Office européen des brevets

(11) EP 4 814 971 A1

(12) EUROPEAN PATENT APPLICATION

(43) Date of publication:
**30.09.2026 Bulletin 2026/40**

(21) Application number: **26163095.8**

(22) Date of filing: **09.03.2026**

(51) International Patent Classification (IPC):
***G16B 20/30*** *(2019.01)* ***G16B 40/20*** *(2019.01)*

(52) Cooperative Patent Classification (CPC):
**G16B 20/30; G16B 40/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **28.03.2025 IN 202521029943**

(71) Applicant: **Tata Consultancy Services Limited Maharashtra (IN)**

(72) Inventors:
- **RANA, Sadhna**
  **751024 Bhubaneswar, Odisha (IN)**
- **SAJEED, Rakshanda**
  **751024 Bhubaneswar, Odisha (IN)**
- **PRADHAN, Swatantra**
  **751024 Bhubaneswar, Odisha (IN)**
- **SRINIVASAN, Rajgopal**
  **500032 Hyderabad, Telangana (IN)**

(74) Representative: **Goddar, Heinz J.**
**Boehmert & Boehmert**
**Anwaltspartnerschaft mbB**
**Pettenkoferstrasse 22**
**80336 München (DE)**

# (54) SYSTEMS AND METHODS FOR PREDICTING IMMUNOGENIC EPITOPES FROM ANTIGEN PROTEIN SEQUENCES

(57) Epitopes identification is a very important problem and has implications in designing vaccines for emerging viruses, bacteria and fight antimicrobial resistance. Traditional methods of epitope mapping are costly, and time consuming. Present disclosure provides a system and a method that processes structural features along with the features from protein language models and apply deep concatenation techniques to combine the features for obtaining a concatenated feature set. A weight for each feature amongst the concatenated features set and a bias for the concatenated features set are learnt to obtain an optimal combination of features set based on which amino acid residues are predicted as one of an epitope or a non-epitope. Further, epitopes are prioritized using a plurality of attention scores capturing a T-B association by using a protein language model, to obtain a set of immunogenic B-cell epitopes.

Antigen sequence
… D I V E Q M …
ESM2 Embeddings
Encoder
Encoder
Encoder
Encoder
1280D vector
Layer 1
Layer 2
Layer 3
Layer 4
transformed vector X
Linear layer
$W_1X + W_2S'$ + bias
CEp or Non-CEp
Structural features

FIG. 3



EP 4 814 971 A1

## Description

CROSS-REFERENCE TO RELATED APPLICATIONS AND PRIORITY

The present application claims priority from Indian application no. 202521029943, filed on March 28, 2025.

TECHNICAL FIELD

The disclosure herein generally relates to Immunoinformatics, and, more particularly, to systems and methods for predicting immunogenic epitopes from antigen protein sequences.

BACKGROUND

B cell epitopes are special regions on an antigen sequence that trigger an immune response by binding to B cells, which then process the antigens. The immune response is highly specific to these regions, making epitopes crucial for the identification and neutralization of pathogens like viruses, bacteria, or other foreign substances. Thus, identification of epitopes is a very important problem and has implications in designing vaccines for emerging viruses, bacteria and fight antimicrobial resistance. Epitope prediction also has value in monoclonal antibody production as prediction of B epitopes is essential to design antibodies that target specific diseases. Traditional methods of epitope mapping are costly, and time consuming, thus computational tools can help reduce cost and time. However, computational prediction of epitopes has been a challenging problem due to low precision of the existing state-of-the-art algorithms.

SUMMARY

Embodiments of the present disclosure present technological improvements as solutions to one or more of the above-mentioned technical problems recognized by the inventors in conventional systems.

For example, in one aspect, there is provided a processor implemented method for predicting immunogenic epitopes from antigen protein sequences. The method comprises receiving, via one or more hardware processors, an antigen protein sequence as an input from a user, wherein the antigen protein sequence comprises a plurality of amino acid residues; extracting, by using a protein language model via the one or more hardware processors, a first set of features for each position of the plurality of amino acid residues comprised in the antigen protein sequence; transforming, by using a first set of linear layers of a Neural Network (NN) via the one or more hardware processors, the first set of features into a learned hidden representation; extracting, via the one or more hardware processors, a second set of features for each position of the plurality of amino acid residues comprised in the antigen protein sequence; concatenating, via the one or more hardware processors, the second set of features and the learned hidden representations to obtain a concatenated feature set; learning, by using a second set of linear layer of the NN via the one or more hardware processors, (i) a weight for each feature amongst the concatenated features set, and (ii) a bias for the concatenated features set, to obtain an optimal combination of features set; predicting, via the one or more hardware processors, the plurality of amino acid residues as one of an epitope or a non-epitope based on the optimal combination of features set to obtain at least one of a set of epitopes and a set of non-epitopes; and prioritizing, via the one or more hardware processors, the set of epitopes using a plurality of attention scores capturing a T-B association by using the protein language model, to obtain a set of immunogenic B-cell epitopes.

In an embodiment, the second set of features comprises at least a contact number, a B-factor, a protrusion index (PI), and a half sphere exposure (HSE).

In an embodiment, the learned hidden representation comprises at least one of a first hidden representation type, and a second hidden representation type.

In another aspect, there is provided a processor implemented system for predicting immunogenic epitopes from antigen protein sequences. The system comprises: a memory storing instructions; one or more communication interfaces; and one or more hardware processors coupled to the memory via the one or more communication interfaces, wherein the one or more hardware processors are configured by the instructions to receive an antigen protein sequence as an input from a user, wherein the antigen protein sequence comprises a plurality of amino acid residues; extract, by using a protein language model, a first set of features for each position of the plurality of amino acid residues comprised in the antigen protein sequence; transform, by using a first set of linear layers of a Neural Network (NN), the first set of features into a learned hidden representation; extract a second set of features for each position of the plurality of amino acid residues comprised in the antigen protein sequence; concatenate the second set of features and the learned hidden representations to obtain a concatenated feature set; learn, by using a second set of linear layer of the NN via the one or more hardware processors, (i) a weight for each feature amongst the concatenated features set, and (ii) a bias for the concatenated features set, to obtain an optimal combination of features set; predict the plurality of amino acid residues

as one of an epitope or a non-epitope based on the optimal combination of features set to obtain at least one of a set of epitopes and a set of non-epitopes; and prioritize the set of epitopes using a plurality of attention scores capturing a T-B association by using the protein language model, to obtain a set of immunogenic B-cell epitopes.

In an embodiment, the second set of features comprises at least a contact number, a B-factor, a protrusion index (PI), and a half sphere exposure (HSE).

In an embodiment, the learned hidden representation comprises at least one of a first hidden representation type, and a second hidden representation type.

In yet another aspect, there are provided one or more non-transitory machine-readable information storage mediums comprising one or more instructions which when executed by one or more hardware processors cause predicting immunogenic epitopes from antigen protein sequences by receiving an antigen protein sequence as an input from a user, wherein the antigen protein sequence comprises a plurality of amino acid residues; extracting, by using a protein language model, a first set of features for each position of the plurality of amino acid residues comprised in the antigen protein sequence; transforming, by using a first set of linear layers of a Neural Network (NN), the first set of features into a learned hidden representation; extracting a second set of features for each position of the plurality of amino acid residues comprised in the antigen protein sequence; concatenating the second set of features and the learned hidden representations to obtain a concatenated feature set; learning, by using a second set of linear layer of the NN via the one or more hardware processors, (i) a weight for each feature amongst the concatenated features set, and (ii) a bias for the concatenated features set, to obtain an optimal combination of features set; predicting the plurality of amino acid residues as one of an epitope or a non-epitope based on the optimal combination of features set to obtain at least one of a set of epitopes and a set of non-epitopes; and prioritizing the set of epitopes using a plurality of attention scores capturing a T-B association by using the protein language model, to obtain a set of immunogenic B-cell epitopes.

In an embodiment, the second set of features comprises at least a contact number, a B-factor, a protrusion index (PI), and a half sphere exposure (HSE).

In an embodiment, the learned hidden representation comprises at least one of a first hidden representation type, and a second hidden representation type.

It is to be understood that both the foregoing general description and the following detailed description are exemplary and explanatory only and are not restrictive of the invention, as claimed.

## BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 depicts an exemplary system for predicting immunogenic epitopes from antigen protein sequences, in accordance with an embodiment of the present disclosure.

FIG. 2 depicts an exemplary high level block diagram of the system for predicting immunogenic epitopes from antigen protein sequences, in accordance with an embodiment of the present disclosure.

FIG. 3 depicts an exemplary high level block diagram of the system of FIGS. 1-2 for predicting immunogenic epitopes from antigen protein sequences, in accordance with an embodiment of the present disclosure.

FIG. 4 depicts an exemplary flow chart illustrating a method for predicting immunogenic epitopes from antigen protein sequences, using the systems of FIGS. 1-3, in accordance with an embodiment of the present disclosure.

FIG. 5 depicts a graphical representation illustrating a comparison of different epitope prediction models with the protein language model implemented by the system of FIGS. 1 through 3, based on Receiver Operating Characteristic (ROC)-curves, in accordance with an embodiment of the present disclosure.

FIG. 6 depicts a graphical representation illustrating a comparison of epitope prediction models based on ROC-curves, in accordance with an embodiment of the present disclosure.

FIG. 7 depicts a graphical representation illustrating distributions of the two extreme cases for the conformational epitope data, in accordance with an embodiment of the present disclosure.

FIG. 8 depicts a graphical representation illustrating distributions of the two extreme cases for the linear epitope data, in accordance with an embodiment of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

B cell epitopes are special regions on an antigen sequence that trigger an immune response by binding to B cells, which then process the antigens. These highly specific immune responses are activated by these distinct regions which make epitopes crucial for the identification and neutralization of pathogens like viruses, bacteria, or other foreign substances (Bahai et al., 2021 - refer "EpitopeVec: linear epitope prediction using deep protein sequence embeddings. Bioinformatics 37, 4517-4525."). Thus, B epitope identification is crucial in the field of immunotherapy and vaccine design.

B-cell epitopes can be identified by different methods including solving a three-dimensional (3D) structure of antigen-antibody complexes, nuclear magnetic resonance spectroscopy, peptide library screening of antibody binding or performing functional assays in which the antigen is mutated, and the interaction antibody-antigen is evaluated (Sanchez-Trincado et al., 2017 - refer "Fundamentals and Methods for T- and B-Cell Epitope Prediction. Journal of Immunology Research 2017, 2680160."). However, these methods are expensive, time-consuming and some require a high level of lab expertise. This is why the development of in silico tools has attracted a lot of attention.

Recently, the field of computational epitope prediction has seen a surge in the use of advanced deep learning methods for improving the accuracy of B epitope predictions. BepiPred3.0 (Clifford et al., 2022 - refer "Improved B - cell epitope prediction using protein language models. Protein Science: A Publication of the Protein Society 31, e4497."), a sequence-based epitope prediction tool has leveraged evolutionary scale modeling, ESM-2 (Lin et al., 2022) to improve the prediction accuracy of both linear and conformational epitope predictions. GraphBepi has shown that using native structures or predicted structures from AlphaFold2 (Jumper et al., 2021 - refer "Highly accurate protein structure prediction with AlphaFold. Nature 596, 583-589.") yields comparable results on conformational epitope prediction task. Similarly, SEMA 2.0 (Ivanisenko et al., 2024 - refer "SEMA 2.0: web-platform for B-cell conformational epitopes prediction using artificial intelligence. Nucleic Acids Research 52, W533-W539.") has shown improved prediction accuracy using ESM and SaProt (Su et al., 2023 - refer "Protein language modeling with structure-aware vocabulary. bioRxiv 2023-10.") (Structure-aware Protein language model). These models have shown that characterization of high-resolution protein complex structure is no longer a major bottleneck for accurately predicting epitopes. These current generation tools using language models and deep learning models such as Alphafold2 have shown significant prediction improvement as compared to other conformational epitope prediction tools from previous generation such as, BepiPred2.0 (Jespersen et al., 2017 - refer "BepiPred-2.0: improving sequence-based B-cell epitope prediction using conformational epitopes. Nucleic Acids Research 45, W24."), Ellipro (Ponomarenko et al., 2008 - refer "ElliPro: a new structure-based tool for the prediction of antibody epitopes. BMC Bioinformatics 9, 514."), Discotope2 (Kringelum et al., 2012 - refer "Reliable B Cell Epitope Predictions: Impacts of Method Development and Improved Benchmarking. PLoS Computational Biology 8, e1002829."), SEPPA3.0 (Zhou et al., 2019 - refer "SEPPA 3.0-enhanced spatial epitope prediction enabling glycoprotein antigens. Nucleic Acids Research 47, W388."), Epitope-3D (da Silva et al., 2022 - refer "epitope3D: a machine learning method for conformational B-cell epitope prediction. Brief Bioinform 23, bbab423."), which use extensive feature engineering and include structural, sequential and evolutionary features of the protein for training the models.

While most of the current approaches incorporate structural information using GNNs or structure aware embeddings, the present disclosure explores combining various explicit structural features along with the features from protein language models. Thus, in the present disclosure, the system and method evaluated if combining protein embeddings with various structural features would lead to a better prediction accuracy. The present disclosure also explored different techniques such as naive concatenation as well as deep transformation and concatenation techniques to combine the protein embeddings and structural features for the protein language model as implemented by the system and the method. The present disclosure observed that on a curated independent test set the combination of structural features with protein embeddings yields better results as compared to a baseline model that used only protein embeddings as features. The protein language model with combined features outperforms other state-of-the-art epitope predictors on most of the metrics. The present disclosure also shows that structural features are also important for predicting linear epitopes. There are studies that have included T-B reciprocity (Zhu et al., 2022) as a feature for improved epitope prediction. The present disclosure shows from the attention analysis that ESM-2 captures T-B reciprocity implicitly as the epitope predictions with high scores are highly attended by the T epitopes.

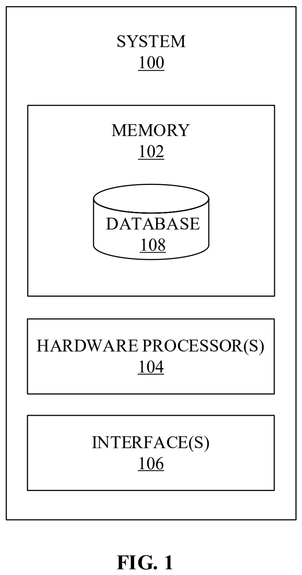

Referring now to the drawings, and more particularly to FIGS. 1 through 8, where similar reference characters denote corresponding features consistently throughout the figures, there are shown preferred embodiments, and these embodiments are described in the context of the following exemplary system and/or method.

FIG. 1 depicts an exemplary system 100 for predicting immunogenic epitopes from antigen protein sequences, in accordance with an embodiment of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the

system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises information a plurality of antigen protein sequences. The database 108 further comprises features extracted from the plurality of antigen protein sequences which are then transformed to a learned hidden representation, and the like. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

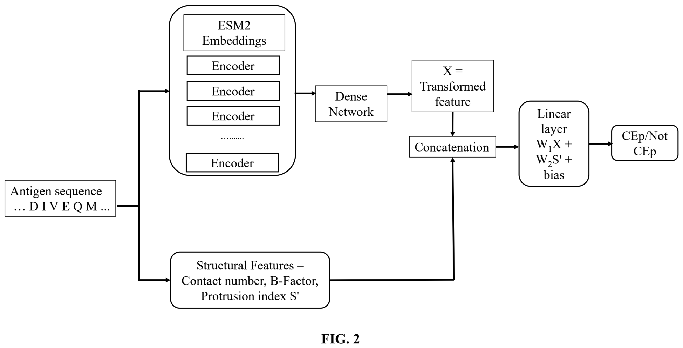

FIG. 2, with reference to FIG. 1, depicts an exemplary high level block diagram of the system 100 for predicting immunogenic epitopes from antigen protein sequences, in accordance with an embodiment of the present disclosure.

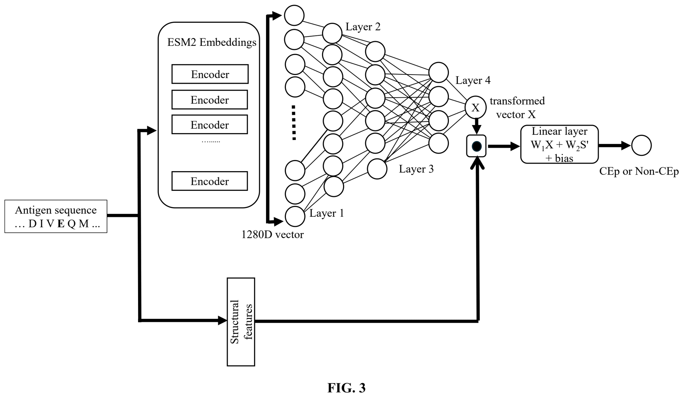

FIG. 3, with reference to FIGS. 1-2, depicts an exemplary high level block diagram of the system 100 for predicting immunogenic epitopes from antigen protein sequences, in accordance with an embodiment of the present disclosure.

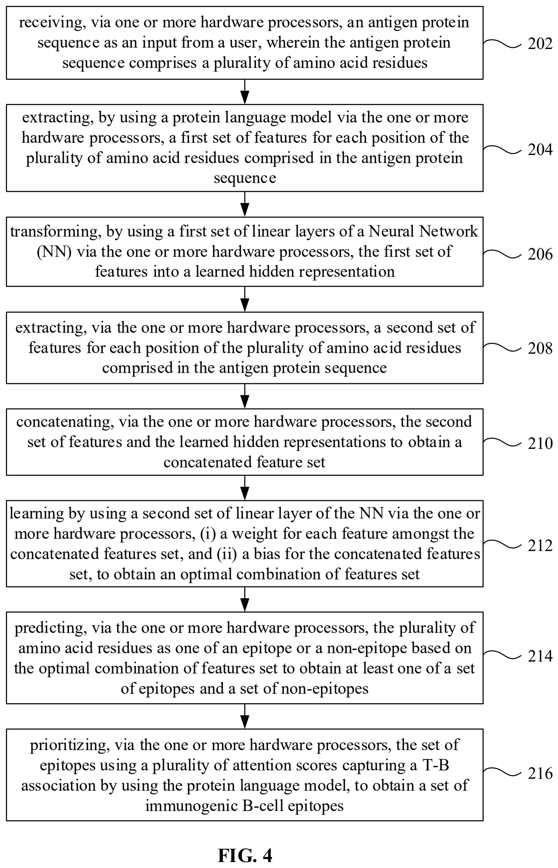

FIG. 4, with reference to FIGS. 1-3, depicts an exemplary flow chart illustrating a method for predicting immunogenic epitopes from antigen protein sequences, using the systems 100 of FIG. 1-3, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, the block diagram of the system 100 depicted in FIGS. 2-3, and the flow diagram as depicted in FIG. 4. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

At step 202 of the method of the present disclosure, the one or more hardware processors 104 receive an antigen protein sequence as an input from a user. The antigen protein sequence comprises a plurality of amino acid residues. For instance, the antigen protein sequence say comprises '...D I V E Q M ...'.

At step 204 of the method of the present disclosure, the one or more hardware processors 104 extract, by using a protein language model, a first set of features for each position of the plurality of amino acid residues comprised in the antigen protein sequence. The protein language model is an Evolutionary Scale Modeling (ESM2) embeddings, in one embodiment of the present disclosure. It is to be understood by a person having ordinary skill in the art or person skilled in the art that the system 100 may implement any other embeddings technique for the extraction of the first set of features and such ESM2 embeddings implemented shall not be construed as limiting the scope of the present disclosure. Examples of other feature extraction tools/embeddings technique include, but are not limited to, ProtBERT, TAPE-BERT, TAPE, and the like. ProtBERT is a pretrained model on protein sequences using a masked language modeling objective. It is based on the BERT model, which is pretrained on a large corpus of protein sequences in a self-supervised fashion. TAPE-BERT (Tasks Assessing Protein Embeddings (TAPE), is a set of five biologically relevant semi-supervised learning tasks spread across different domains of protein biology.

At step 206 of the method of the present disclosure, the one or more hardware processors 104 transform, by using a first set of linear layers of a Neural Network (NN), the first set of features into a learned hidden representation. The learned hidden representation comprises at least one of a first hidden representation type, and a second hidden representation type. For instance, the first hidden representation type is a low-dimensional learned hidden representation, and the second hidden representation type is a high-dimensional learned hidden representation. In other words, the first set of features are transformed into either the low-dimensional learned hidden representation, or the high-dimensional learned hidden representation. In the present disclosure, the system 100 transformed the first set of features into the low-dimensional learned hidden representation. It is to be understood by a person having ordinary skill in the art or person skilled in the art that such transformation shall not be construed as limiting the scope of the present disclosure. The Neural Network is a dense network, in one embodiment of the present disclosure. The Neural Network comprises 4 hidden layers as depicted in FIG. 3. A first layer (layer 1) takes 'm' dimensional vector (e.g., 1280-dimensional vector) as input and outputs a 'x' dimensional vector (e.g., say 512-dimensional vector). The second hidden layer (layer 2) takes the 'x' dimensional vector

(e.g., 512-dimensional vector) and outputs a 'y' dimensional vector (e.g., say 250-dimensional vector). Then the third hidden layer (layer 3) takes 'y' dimensional vector (e.g., 250-dimensional vector) and outputs 'z' dimensional vector (e.g., 10-dimensional vector) and the third hidden layer (layer 4) takes 'z' dimensional vector (e.g., 10-dimensional vector) and finally gives a p-D transformed vector (e.g., 1-D transformed vector). The p-D transformed vector is the learned hidden representation, in one embodiment of the present disclosure. For regularization, the system 100 and the method of the present disclosure added a dropout of 0.2 between layers 1 and 2 and also between layers 2 and 3. Activation functions (ReLU) were also added by the system 100 between each layer to introduce non-linearity.

At step 208 of the method of the present disclosure, the one or more hardware processors 104 extract a second set of features for each position of the plurality of amino acid residues comprised in the antigen protein sequence. The second set of features comprises structural features, in one embodiment of the present disclosure. More specifically, the structural features are contact number (Yuan, 2005 - refer "Better prediction of protein contact number using a support vector regression analysis of amino acid sequence. BMC Bioinformatics 6, 248."), B-factor (Ren et al., 2014 - refer "Tertiary structure-based prediction of conformational B-cell epitopes through B factors. Bioinformatics 30, 1264-273."), Protrusion Index (PI) (Xia et al., 2010 - refer "APIS: accurate prediction of hot spots in protein interfaces by combining protrusion index with solvent accessibility. BMC Bioinformatics 11, 174.") and Half Sphere Exposure (HSE) (Sweredoski and Baldi, 2008 - refer "PEPITO: improved discontinuous B-cell epitope prediction using multiple distance thresholds and half sphere exposure. Bioinformatics 24, 1459-1460."). The contact number was computed as the number of residues whose $C_{bet\alpha}$ atoms were present within a distance threshold (10A) of the residue of interest. B-factor is an atomic attribute recorded during the process of crystallizing the protein complex. It characterizes the weakened dispersion of the neutrons due to random motion during the process. This feature was derived from the Protein Data Bank (PDB) (Burley et al., 2017 - refer "The Single Global Macromolecular Structure Archive. Methods Mol Biol 1607, 627-641.") files of the final chosen complexes. The B-factor was identified for each atom, and the system 100 calculated the average B-factor for the atoms of the residue of interest. PI is a geometric structural feature which estimates the extent to which the residue is protruding from the surface of the protein complex. The number of heavy atoms ($N_{atoms}$) within a fixed distance R (10A) was calculated. Then, $N_{atoms}$ is multiplied by the mean atomic volume which is the volume occupied by the protein within the sphere, $V_{\text{int}}$ which is $20.1 \pm 0.9Å^3$. The difference between the volume of the sphere and $V_{int}$ is given by $V_{ext}$. The protrusion index is then calculated as $V_{exe}/V_{int}$. HSE measures the solvent exposure of the protein and is calculated by counting the amino acid neighbors within the two half spheres of a certain radius (usually 12Å) around the amino acid residue. This shows how buried the residue is in the protein. For this, the BioPDB ("Bio.PDB package - Biopython 1.75 documentation,"n.d.) module HSE was used. The HSE is calculated for the residues of the known protein chain around a radius of the requirement.

At step 210 of the method of the present disclosure, the one or more hardware processors 104 concatenate the second set of features and the learned hidden representations to obtain a concatenated feature set. The system 100 implemented a naive vector concatenation, and deep transformation and concatenation techniques as known in the art to combine features and obtain the informative representation of an epitope (e.g., the concatenated feature set). The learned hidden representation (with outputs of intermediate layers - layer 1, layer 2, and so on), the second set of features, and the concatenated feature set as outputs of steps 206, 208, and 210 respectively are shown in Table 1 by way of the following exemplary values, and such values shall not be construed as limiting the scope of the present disclosure.

Table 1

| pdb _id | chai n | seq | pos | ami no acid | inpu t_fe atur es (128 0 d) | Lay er 1 outp ut (512 d) | Lay er 2 outp ut (250 d) | Lay er 3 outp ut (10 d) | Lay er 4 outp ut (1 d) | B-fact or | PI | HS E_U | HS E_D | Con cate nate d Feat ures |
|---|---|---|---|---|---|---|---|---|---|---|---|---|---|---|
| 1BZ Q | A | KE T... DA SV | 1 | K | - 0.02 984 130 196 273 32, - 0.18 754 306 435 585, - 0.06 513 635 069 131 85, . .., 0.03 454 907 983 541 48, - 0.17 576 026 916 503 9, - 0.19 797 348 976 135 3 | 0.05 885 810 405 015 94, - 0.06 572 717 428 207 39, - 0.39 613 455 533 981 3, ... , - 0.01 303 310 599 178 07, - 0.02 078 161 574 900 15, - 0.02 585 305 087 268 35 | 0.13 239 337 503 910 1, 0.13 561 123 609 542 8, 0.02 919 204 160 571 09, . .., 0.03 923 165 425 658 2, - 0.15 552 471 578 121 2, - 0.05 296 988 785 266 87 | 0.03 297 366 574 406 62, - 0.30 240 499 973 297 1, ... , 0.18 375 387 787 818 9, - 0.33 313 873 410 224 9 | 0.24 609 921 872 615 8 | 48.0 944 | 51.0 99 | 0 | 6 | 0.24 609 921 872 615 8, 48.0 944, 51.0 99, 0, 6 |
| 1BZ Q | A | KE T... DA SV | 2 | E | 0.05 592 072 755 098 34, - 0.06 932 150 572 538 37, - 0.12 677 329 778 671 3, ... , 0.16 656 829 416 751 9, - 0.12 051 775 306 463 2, - 0.04 335 975 274 443 62 | 0.04 204 004 257 917 4, - 0.07 263 864 576 816 55, - 0.05 449 500 307 440 75, . .., 0.13 351 285 457 611 1, - 0.06 226 564 943 790 43, 0.09 517 626 464 366 91 | 0.03 711 090 236 902 23, - 0.27 075 165 510 177 6, - 0.51 871 567 964 553 8, ... , - 0.08 579 538 017 511 36, 0.15 108 135 342 598, - 0.00 345 930 201 001 46 | - 0.03 303 000 330 924 98, 0.02 218 607 068 061 82,. .., 0.01 961 315 236 985 68,- 0.09 445 358 812 808 99 | - 0.01 401 023 752 987 38 | 42.1 366 7 | 28.7 710 7 | 10 | 9 | - 0.01 401 023 752 987 38, 42.1 366 7, 28.7 710 7, 10, 9 |
| 6R7 T | B | DP V.. ER AQ | 179 | A | 0.00 123, 0.05 933, - 0.07 143, ..., - 0.20 292, - 0.04 431 3, 0.04 182 3 | - 0.08 204, - 0.17 817, 0.16 584, ..., 0.06 090, 0.06 273, - 0.02 523 | - 0.14 024, - 0.03 358 9, - 0.19 918 5, ... , 0.21 581 3, 0.01 269 93, 0.01 978 7 | - 0.02 906, 0.21 819, ..., 0.04 049 7, - 0.16 167 0 | 0.25 335 538 387 298 6 | 74.4 920 | 28.7 710 | 5 | 4 | 0.25 335 538 387 298 6, 74.4 920, 28.7 710, 5, 4 |
| 6R7 T | B | DP V..ER AQ | 180 | Q | 0.04 624, 0.03 113, - 0.33 917, ..., 0.09 682, - 0.10 497, 0.02 026 72 | 0.05 901 4, -0.24 86, - 0.04 067, ..., - 0.14 277, 0.26 075, 0.02 330 3 | - 0.10 931 525, 0.04 964 73, - 0.10 160, ..., 0.15 541, 0.19 270, 0.10 536 1 | 0.08 151 184 022 426 6, 0.09 178 014 844 655 99, 0.26 443 034 410 476 7, ... , - 0.18 279 743 194 580 1, - 0.00 945 368 036 627 76, - 0.20 118 762 552 738 2 | 0.02 758 794 464 170 93 | 88.6 955 | 33.7 329 | 4 | 3 | 0.02 758 794 464 170 93, 88.6 955 5, 33.7 329, 4, 3 |

At step 212 of the method of the present disclosure, the one or more hardware processors 104 learn, by using a second set of linear layer of the NN, (i) a weight for each feature amongst the concatenated features set, and (ii) a bias for the concatenated features set, to obtain an optimal combination of features set. More specifically, as depicted in FIG. 3, the system 100 learns different weights as well as common representations for protein embeddings and structural features before concatenation. To achieve this, the ESM-2 embeddings were transformed through the first 3 layers of the neural network, then the transformed feature vector was concatenated with different combinations of structural features which is then given as input to the final linear layer of the NN. In the present disclosure, to learn the weights of the for each feature amongst the concatenated features set the system 100 implemented a 5-fold cross validation technique with an outer testing loop and an inner validation loop for training. In the outer loop, to obtain reliable and stable predictions and to avoid over-fitting and biases in the training set, the system 100 split 901 complexes into five random sets. Four sets out of five were used for training. This training set was split into a ratio of 85:15 and used for training model and hyper parameter optimization. This step is performed 'p' times (e.g., 5 times) so that the system 100 had p (e.g., 5) trained models and p (e.g., 5) test sets. The metrics from these five test sets corresponding to their models were averaged to get the final metric. The system 100 performed extensive hyperparameter tuning by exploring the different number of layers, number of nodes in each layer, type of optimizer, learning rate in order to ensure a good model (Table 2). Regularization techniques such as dropout layers and early stopping were also implemented to mitigate the over-fitting issues during the learning process of the protein language model.

At step 214 of the method of the present disclosure, the one or more hardware processors 104 predict the plurality of amino acid residues as one of an epitope or a non-epitope based on the optimal combination of features set to obtain at least one of a set of epitopes (e.g., conformational epitopes) and a set of non-epitopes (e.g., non-conformational/linear epitopes) as shown in FIG. 3.

At step 216 of the method of the present disclosure, the one or more hardware processors 104 prioritize the set of epitopes using a plurality of attention scores capturing a T-B association by using the protein language model, to obtain a set of immunogenic B-cell epitopes. The set of epitopes are prioritized to obtain the set of immunogenic B-cell epitopes for designing antigens for vaccines, in one embodiment of the present disclosure. An attention analysis was carried out by the system 100 to check if T-B cell epitope association was captured by the protein language model (e.g., the ESM-2 embeddings) implicitly and helped in predicting immunogenic B-cell epitopes. For validating the T-B association analysis was performed, the system 100 shortlisted 156 antigens from IEDB which had both T-cell and B-cell epitope annotations. This analysis was restricted to known (true) epitopes only. The system 100 extracted the attention matrices for these antigens from ESM-2. These matrices are composed of the attention scores, where an attention score refers to the numerical values assigned to different amino acids of protein sequence data in a transformer based pre-trained language model (PLM) architecture, which are used to compute the importance of each amino acids during the protein language model's processing.

**Results and Experiments**

**Training and test data for conformational epitopes**

The IEDB-3D (Ponomarenko et al., 2011) full-bcr assay dataset was the basis for training and evaluation process by the system 100 (http://www.iedb.org/database_export_v3.php). The dataset consisted of 4554 entries with antigen information, antibody information, host information and the experimental information. To ensure a good dataset for training, the system 100 retained complexes with resolution better than 3Å and the entries with only protein epitopes. This reduced the number of data points from 4554 to 1827. The system 100 also removed the antigen sequences with length less than 60 amino acids. For an unbiased data, a redundancy check was done for the antigen sequences at 50% identity threshold using MMSeqs2 (Steinegger and Soding, 2017 - refer "MMseqs2: sensitive protein sequence searching for the analysis of massive data sets.") clustering tool. From each cluster, only the protein sequence containing the maximum number of residues in the epitope region was retained, reducing the number of antigen sequences to 901. Of these 901 antigen sequences the positive datapoints were those which were experimentally characterized as epitope residues and the remaining as non-epitopes or negative datapoints. These 901 complexes were segregated into 5 test groups with 180 antigens in each. To compare with known epitope prediction tools, BepiPred2.0 (Jespersen et al., 2017), BepiPred3.0 (Clifford et al., 2022), DiscoTope2.0 (Kringelum et al., 2012), DiscoTope3.0 (Hoie et al., 2024) and SEMA2.0 (Ivanisenko et al., 2024) the system 100 checked the similarity of their training sets with 5 test set groups at 50% sequence identity thresholds. For each group the system 100 balanced the positive and negative datapoints in the respective train and test sets. The system 100 evaluated the models on a non-redundant independent test data created from SabDAb, the Dset_anti antigen dataset (Hou et al., 2021 - refer "SeRenDIP-CE: sequence-based interface prediction for conformational epitopes. Bioinformatics 37, 3421-3427.") consisting of 280 antigen-antibody complexes. After executing redundancy checks (25% identity cut off) with the training sets of all the predictors that were used for model comparison in this study, 46 antigen-antibody complexes were left in the final independent evaluation dataset.

**Training and test data for linear epitopes**

The system 100 extracted the linear B-cell epitope data from IEDB. The system 100 selected antigens that were annotated with the Uniprot ID so that they could be mapped to a PDB structure. There were 1184 datapoints with the parent protein information. The system 100 also followed the usual filtration steps of removing antigens with resolutions less than 3Å and redundant (25% identity threshold). Finally, 438 antigens were obtained which were divided the data into train, validation and test sets consisting of 339, 27 and 72 antigens respectively.

**Conformational epitope prediction**

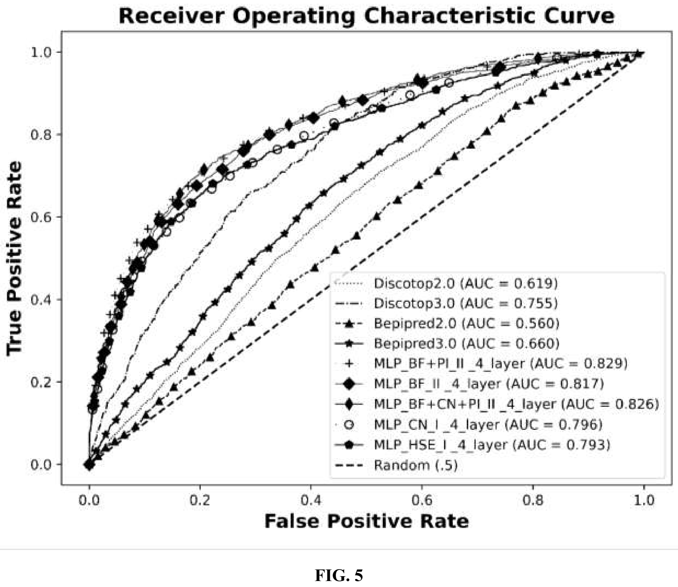

The system 100 trained various models on conformational epitope data using the FIG. 2 and FIG. 3 architectures and various feature combinations. The system 100 evaluated the various trained models on the independent test set consisting of 46 antigens. For the final prediction for each data point the system 100 calculated the mean of the predicted scores from the 5 models which were trained using the nested 5-fold cross validation techniques. The system 100 of FIG. 3, that used feature transformation and concatenation technique performed better than a simple architecture comprising a naive concatenation of the structural features and the ESM-2 embeddings in the input layer. The concatenated features were passed through a neural network that classifies residues as epitope or non-epitope. The system 100 of FIG. 3 that uses ESM-2 features along with structural features PI and B-factor performed best with AUROC of 0.829 as shown in FIG. 5. More specifically, FIG. 5, with reference to FIGS. 1 through 4, depicts a graphical representation illustrating a comparison of different epitope prediction models with the protein language model implemented by the system 100 of FIGS. 1 through 3, based on Receiver Operating Characteristic (ROC)-curves, in accordance with an embodiment of the present disclosure.

The models of the system 100 fared better than other conventional methods due to the increase in training data as well as unique feature combinations. In order to do a fair comparison, the system 100 trained and tested the models of the system 100 of the present disclosure on the training and test data of BepiPred3.0 and GraphBepi. The model of the system 100 performed better than BepiPred3.0 based on AUROC as shown in Table 2 and is at par with GraphBepi as shown in Table 3. Different conformational epitope prediction models developed, and their metrics are shown in Table 2.

Table 2 (Performance comparison of our best performing models with BepiPred3.0. These models have been trained and tested on BepiPred3.0 train and test data. (BF: B-factor, CN: Contact number, PI: Protrusion index, HSE: Half-sphere exposure)

| Model | AUROC | AUPRC | MCC | F1-score |
|---|---|---|---|---|
| BepiPred 3.0 | 0.665 | 0.399 | 0.187 | 0.383 |
| MLP | 0.704 | 0.322 | 0.214 | 0.376 |
| MLP_BF_II | 0.742 | 0.344 | 0.261 | 0.402 |
| MLP_BF+PI_II | 0.507 | 0.155 | 0.087 | 0.300 |
| MLP_CN+BF+PI_II | 0.733 | 0.340 | 0.238 | 0.382 |

Table 3 (Comparison of the best performing models of the system 100 trained on Graphbepi train data. (BF: B-factor, CN: Contact number, PI: Protrusion index, HSE: Half-sphere exposure)

| Model | AUROC | AUPRC | MCC | F1-score |
|---|---|---|---|---|
| Graphbepi | 0.751 | **0.261** | 0.232 | 0.310 |
| CEP | 0.675 | 0.157 | 0.141 | 0.227 |
| MLP_BF_II | 0.713 | 0.185 | 0.189 | 0.248 |
| MLP_CN+BF+PI_II | 0.690 | 0.148 | 0.167 | 0.240 |
| **MLP_BF+PI_II** | **0.753** | **0.225** | **0.232** | **0.300** |
| MLP_HSE_II | 0.663 | 0.141 | 0.036 | 0.065 |

The best model of the system 100 was also compared with ClusPro AbEMap (Desta et al., 2023 - refer "The ClusPro AbEMap web server for the prediction of antibody epitopes. Nature protocols 18, 1814.") tool which is used for epitope prediction. It was observed that AbEMap performs better than most of the popular epitope prediction tools like

SEPPA, BEPro and EpiPred (Krawczyk et al., 2014 - refer "Improving B-cell epitope prediction and its application to global antibody-antigen docking. Bioinformatics 30, 2288."). Thus, the models implemented by the system 100 were evaluated with the test set used by AbEMap for observing their performance. The system 100 and the method conducted the experiments to ensure that the antigen sequences were not more than 25% identical to the training set. It was seen that while AbEMap obtained an average ROC AUC score of 0.738, the model of the system 100 trained on the IEDB data scored 0.81. In order to test how structure aware SaProt model fares against ESM-2, the system 100 also trained models by extracting structure aware features from SaProt. The system 100 used SaProt embeddings as known in the art to extract features for protein sequences in the training and test set. The best model architectures were used for training the models. The results show (Table 4) that AUROC of the model using only SaProt embeddings (0.655) lag behind when the combined embeddings with structural features (0.674) are used in this study. From these results it can be inferred that the structural features considered by the system 100 and the method of the present disclosure are significant in characterizing epitope residues better.

Table 4: Models trained on LEP (LEP_MLP_single_fold_4_layer models)/CEP (CEP_MLP_5_fold_4_layer models) data and tested on LEP data

| **Model name** | **Acc** | **Pre** | **Rec** | **AUROC** | **Bal_acc** | **TPR** | **TNR** | **FPR** | **FNR** | **AUPRC** | **f1-score** |
|---|---|---|---|---|---|---|---|---|---|---|---|
| LEP_MLP_single_fold_4_layer_BF_II | 0.69 | 0.90 | 0.62 | 0.74 | 0.73 | 0.62 | **0.85** | **0.15** | 0.37 | 0.86 | 0.74 |
| LEP_MLP_single-fold_4_layer_BF+PI_II | 0.78 | 0.91 | **0.78** | 0.75 | 0.77 | **0.78** | 0.76 | 0.24 | **0.21** | 0.88 | **0.84** |
| LEP_MLP_single-fold_4_layer_CN_BF+PI_II | **0.74** | **0.90** | 0.71 | **0.77** | **0.76** | 0.71 | 0.81 | 0.18 | 0.29 | **0.87** | 0.80 |
| CEP_MLP_5-fold_4_layer_BF_II | 0.58 | 0.90 | **0.35** | 0.64 | 0.64 | **0.35** | 0.94 | 0.05 | **0.64** | 0.76 | **0.50** |
| CEP_MLP_5-fold_4_layer_BF+PI_II | 0.54 | **0.90** | 0.22 | **0.60** | **0.59** | 0.22 | **0.96** | **0.03** | 0.77 | 0.69 | 0.36 |
| CEP_MLP_5-fold_4_layer_CN_BF+PI_II | **0.55** | 0.90 | 0.28 | 0.61 | 0.62 | 0.28 | 0.95 | 0.04 | 0.71 | 0.73 | 0.43 |
| BepiPred2.0 | 0.50 | 0.52 | 0.70 | 0.47 | 0.48 | 0.70 | 0.26 | 0.73 | 0.30 | 0.52 | 0.60 |
| BepiPred3.0 | 0.49 | 0.57 | 0.25 | 0.49 | 0.51 | 0.25 | 0.77 | 0.22 | 0.74 | 0.55 | 0.34 |
| Epidope | 0.55 | 0.56 | 0.78 | 0.56 | 0.53 | 0.78 | 0.27 | 0.72 | 0.21 | 0.60 | 0.65 |

Table 4 shows the different metrics for the models trained on linear epitope data and tested on linear epitope test data which have been prefixed as 'LEP_MLP', and metrics of models trained on conformational epitope data and tested on linear epitope test data which have been prefixed as 'CEP_MLP'. The system 100 has compared these models with the prediction tools BepiPred2.0 and BepiPred3.0, and EpiDope tools.

**Linear epitope prediction:** Most of the conformational epitope prediction tools use structural features, however linear epitope predictors have so far mostly used sequence based features except Ellipro that used PI. It has been shown that structural features can be important for predicting linear epitopes as well (Barlow et al., 1986 - refer "Continuous and discontinuous protein antigenic determinants. Nature 322, 747-748."). Thus, the system 100 trained a linear epitope predictor as known in the art using the same set of combination features (ESM-2, PI, CN, HSE, B-factor) as the system 100 did for the conformational epitopes. The linear epitope train, validation and test data were prepared accordingly. Similar

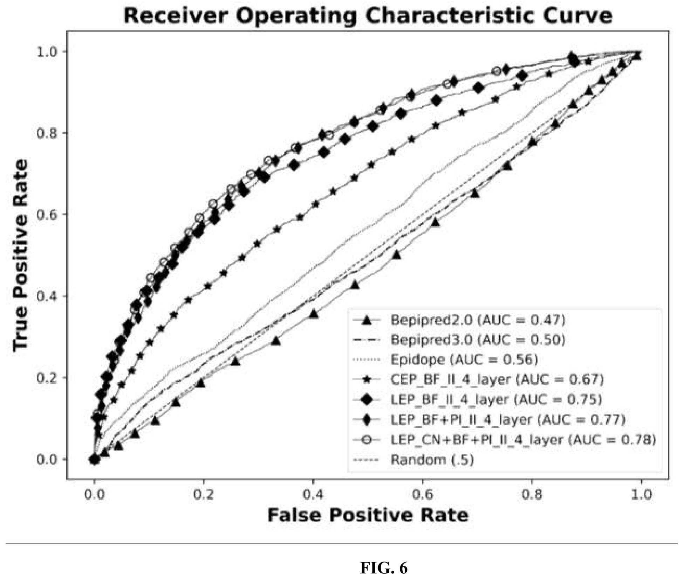

architectures and feature combinations were used as done for the conformational epitope models. As shown in Table 3, the best linear model that uses deep combination of ESM-2 features with structural features CN, BF and PI, has AUROC score of 0.77 and outperforms BepiPred3.0 and Epidope (linear epitope predictor) that have AUROC of 0.49 and 0.56 respectively. The system 100 also tested the performance of the best conformational epitope models trained on the conformational data on the linear epitope test data. As seen in Table 4 and FIG. 6, models trained on linear epitope data perform slightly better on the linear test dataset than the ones trained on the conformational data. FIG. 6, with reference to FIGS. 1 through 5, depicts a graphical representation illustrating a comparison of epitope prediction models based on ROC-curves, in accordance with an embodiment of the present disclosure. The models were tested on linear epitope test set. LEP: models were trained on LEP data and CEP: models trained on CEP data. **ESM embeddings capture T-B reciprocity:** Studies have shown that B cells are helped by T cells in antibody production (Lanzavecchia, 1985 - refer "Antigen-specific interaction between T and B cells. Nature 314, 537-539."). It has been observed that the binding of antibodies also influences the process of antigen processing and presentation. This was seen based on the relative positions of the T-cell epitopes and B-cell epitopes on the antigen. The theory of immunodominance states that the relative positions of the T and B cell epitopes on the antigen influences its immunodominance (Biavasco and De Giovanni, 2022 - refer "The Relative Positioning of B and T Cell Epitopes Drives Immunodominance. Vaccines 10, 1227."). The selection of B cell receptors with higher affinity is influenced and aided by the CD4 T helper cells in an epitope environment. This process is known as T-B reciprocity (Zhu et al., 2022 - refer "Language models of protein sequences at the scale of evolution enable accurate structure prediction."). In simpler terms, the immunogenicity of those B-cell epitopes is enhanced which have T-cell epitopes present nearby.

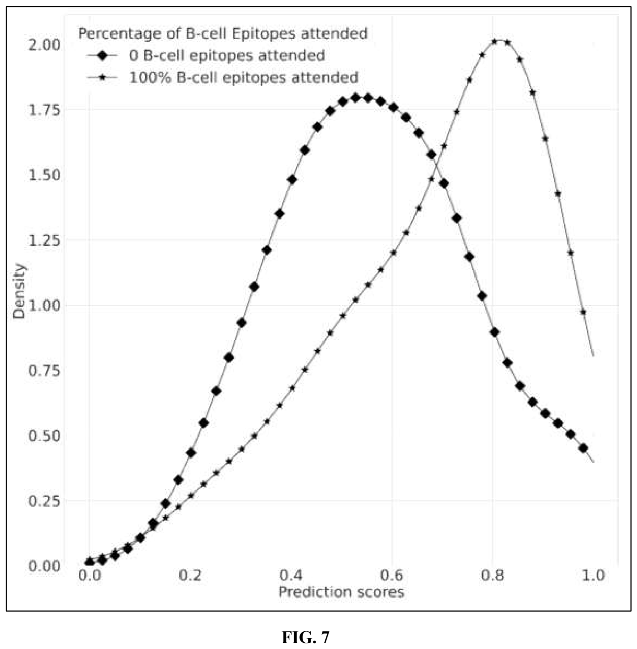

As mentioned above, the system 100 carried out an attention analysis to check if T-cell epitope proximity was captured by ESM-2 implicitly and helped in predicting immunogenic B-cell epitopes. For the analysis, the system 100 shortlisted 156 antigens from IEDB which had both T-cell and B-cell epitope annotations. Thus, this analysis was restricted to known (true) epitopes only. We extracted the attention matrices for these antigens from ESM-2. These matrices are composed of the attention scores which are the numerical values underscoring the importance of each residue in the protein sequence and represent the contextual dependency between the residues in the sequence. ESM-2 (650M) model consisted of 33 transformer encoder layers each having 20 attention heads. These multiple attention heads extend the model's capability to focus on different residue positions and their relevance with each other in the sequence and help in learning the multiple representation subspaces for the input sequence. Thus, the last layer of the ESM-2 model was accessed to take the attention matrices from the 20 attention heads for each antigen sequence. It is to be understood by a person having ordinary skill in the art or person skilled in the art that such configuration of the protein language model as ESM2 (or ESM-2) shall not be construed as limiting the scope of the present disclosure. For the known T-cell epitope residues the system 100 acquired the attention scores and averaged them across all the 20 attention heads. For the known T-cell epitope residue positions the corresponding attention score vector was obtained. These attention score vectors provide the information about the top attended residues for each T-cell epitope residue position. The T-cell epitope residue positions attention score vectors were sorted in a specific format (e.g., say descending manner and such format shall not be construed as limiting the scope of the present disclosure) and from these sorted vectors the system 100 obtained the top residues whose attention scores sum up to 0.5 as these positions were considered as highly attended by the ESM-2 when looking at the T-cell epitopes. This was shown in the form of percentage occurrence with respect to the total known B-cell epitopes for the respective antigen sequence. The distribution of the frequency of the B-cell epitopes attended by the T-cell epitopes for the 156 PDB complexes was also observed. Majority of the antigens used in this analysis had B-cell epitopes that were highly attended by the T-cell epitopes. It was observed that in 116 antigens at least one B epitope was attended by the T epitopes, in 18 antigens all the known B-cell epitopes were highly attended by the T-cell epitopes. There were 40 cases where none of the B-cell epitopes were attended by any T-cell epitope. To examine the difference between these two extremities, the system 100 predicted the scores for the B-cell epitopes of these 58 sequences and ran a statistical t-test ("ttest_ind - SciPy v1.14.1 Manual," n.d.) on the predicted scores for these two sets. The calculated p-value for this test was seen to be 0.03 which shows that there is a significant difference between the representation of the scores between these two data samples (e.g., refer FIG. 7). More specifically, FIG. 7, with reference to FIGS. 1 through 6, depicts a graphical representation illustrating distributions of the two extreme cases for the conformational epitope data, in accordance with an embodiment of the present disclosure. The area under the 100% B-cell epitope attended curve represents the distribution of the predicted scores for the sequences where all the B-cell epitopes were attended to by the T-cell epitopes. The area under the 0% B-cell epitope curve represents the distribution of the predicted scores for the sequences where none of the B-cell epitopes were attended to by the T-cell epitopes. From these observations the system 100 inferred that the protein large language models are able to capture some form of T-cell and B-cell epitope reciprocity, which can be seen with the help of attention. This finding is crucial as it allows the researcher to filter high scoring immunogenic (owing to T-B reciprocity) sequences for antigen design.

This analysis was also done for the linear B-cell epitopes. For this the system 100 obtained 254 antigen sequences for which both the B-cell and T-cell epitope information was known. By performing the same attention analysis it was observed that for certain sequences all the known B-cell epitopes were highly attended by the T-cell epitopes. Such

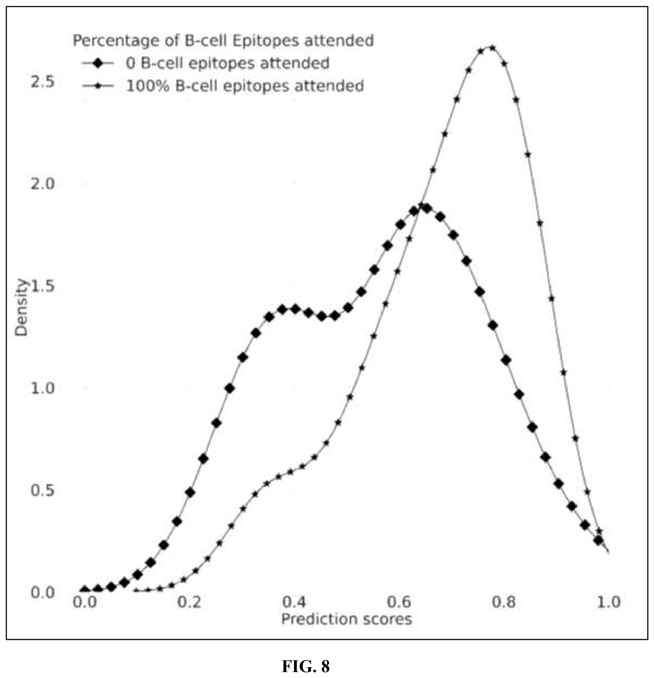

cases were seen to be 31 out of the 254 antigens. There were 35 cases where none of the B-cell epitopes were attended by any T-cell epitope. To examine the difference between these two extremities, the system 100 predicted the attention scores for the B-cell epitopes of these 58 sequences and executed a statistical t-test on the predicted scores for these two sets. The calculated p-value for this test was seen to be 0.007 which shows that there is a significant difference between the representation of the scores between these two data samples. FIG. 8, with reference to FIGS. 1 through 7, depicts a graphical representation illustrating distributions of the two extreme cases for the linear epitope data, in accordance with an embodiment of the present disclosure. The area under the 100% B-cell epitope attended curve represents the distribution of the predicted scores for the sequences where all the B-cell epitopes were attended to by the T-cell epitopes. The area under the 0% B-cell epitope attended curve represents the distribution of the predicted scores for the sequences where none of the B-cell epitopes were attended to by the T-cell epitopes.

**Leveraging attention for prediction of immunogenic epitopes**

For the T-B reciprocity captured by attention, the system 100 looked at the Diphtheria toxoid protein chain of 1MDT. Here the known B-cell epitopes are seen in the regions 255-260, 381-394, 395-403, 452-458, 465-475 and the T-cell epitope regions are 271-290, 351-371, 411-430, 431-450 (Biavasco and De Giovanni, 2022). The system 100 calculated attention scores for chain A (diphtheria toxoid) from ESM-2 and obtained top 50% highly attended positions in the antigen with respect to the T-cell epitope positions. The analysis showed that the B epitopes 386, 395, 399, 452-458, 401-403, 465-466 are highly attended by the T-cell epitopes and are also predicted with high confidence (score high) by our best model. The proximity of highly attended B and T epitopes was visualized by PyMol.

In the present disclosure, the system 100 and the method have shown that epitope predictions from the ESM-2 model can be improved by combining structural features with the ESM-2 embeddings. The system 100 further demonstrated that the feature combination techniques can have impact on the prediction accuracy of the models. The system 100 implemented feature combination techniques wherein the system 100 involved converting ESM-2 embeddings (1280 features) to a single transformed feature via layers of neural network. This transformed feature along with structural features is again passed through a linear layer to get a final weighted representation. From the analysis, the system 100 gave improved predictions and the improvement in both conformational and linear epitope prediction was observed by combining ESM-2 embeddings with structural features. This is significant especially for linear epitopes as the majority of algorithms use only sequence based features for linear epitope prediction except Ellipro (Ponomarenko et al., 2008 - refer "ElliPro: a new structure-based tool for the prediction of antibody epitopes. BMC Bioinformatics 9, 514.") that using protrusion index. The best models (both linear and conformational epitope) were compared favorably with other state-of-the-art methods. It has been observed that models with ESM-2 embeddings along with B-factor and PI as structural features are best, underscoring the importance of protein flexibility and shape for predicting epitopes. The system 100 also shows that T-B reciprocity is captured by ESM-2 and helps in prediction of high scoring immunogenic epitopes. Though a large portion of predicted B epitopes are highly attended by T epitopes, there are also other high scoring B epitopes that are not attended by T epitopes at all. Therefore, attention analysis can be used to differentiate high scoring immunogenic B epitopes that are highly attended by T epitopes from high scoring antigenic B epitopes.

While most of the current approaches incorporate structural information using Graph Neural Networks (GNNs) or structure aware embeddings, the system 100 explored combining various explicit structural features along with the features from protein language models. Thus, in the present disclosure, the system 100 evaluated combining protein embeddings with various structural features for a better prediction accuracy. Through experiments, the system 100 observed that on a curated independent test set the combination of structural features with protein embeddings yielded better results as compared to a baseline model that used only protein embeddings as features. The model of the present disclosure with combined features outperforms other state-of-the-art epitope predictors as well. The system 100 also showed that ESM captures B-T reciprocity that has relevance in immunogenicity of epitopes and a large percentage of predicted high scoring epitopes are highly attended by T epitopes. Based on relationship of B and T epitopes as discerned by attention mechanism, best immunogenic epitopes can be prioritized for designing antigens for vaccines.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-program-

mable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

**1.** A processor implemented method, comprising:

receiving, via one or more hardware processors, an antigen protein sequence as an input from a user, wherein the antigen protein sequence comprises a plurality of amino acid residues (202);
extracting, by using a protein language model via the one or more hardware processors, a first set of features for each position of the plurality of amino acid residues comprised in the antigen protein sequence (204);
transforming, by using a first set of linear layers of a Neural Network (NN) via the one or more hardware processors, the first set of features into a learned hidden representation (206);
extracting, via the one or more hardware processors, a second set of features for each position of the plurality of amino acid residues comprised in the antigen protein sequence (208);
concatenating, via the one or more hardware processors, the second set of features and the learned hidden representations to obtain a concatenated feature set (210);
learning, by using a second set of linear layer of the NN via the one or more hardware processors, (i) a weight for each feature amongst the concatenated features set, and (ii) a bias for the concatenated features set, to obtain an optimal combination of features set (212);
predicting, via the one or more hardware processors, the plurality of amino acid residues as one of an epitope or a non-epitope based on the optimal combination of features set to obtain at least one of a set of epitopes and a set of non-epitopes (214); and
prioritizing, via the one or more hardware processors, the set of epitopes using a plurality of attention scores capturing a T-B association by using the protein language model, to obtain a set of immunogenic B-cell epitopes (216).

2. The processor implemented method as claimed in claim 1, wherein the second set of features comprises at least a contact number, a B-factor, a protrusion index (PI), and a half sphere exposure (HSE).

3. The processor implemented method as claimed in claim 1, wherein the learned hidden representation comprises at least one of a first hidden representation type, and a second hidden representation type.

4. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive an antigen protein sequence as an input from a user, wherein the antigen protein sequence comprises a plurality of amino acid residues;
extract, by using a protein language model, a first set of features for each position of the plurality of amino acid residues comprised in the antigen protein sequence;
transform, by using a first set of linear layers of a Neural Network (NN), the first set of features into a learned hidden representation;
extract a second set of features for each position of the plurality of amino acid residues comprised in the antigen protein sequence;
concatenate the second set of features and the learned hidden representations to obtain a concatenated feature set;
learn, by using a second set of linear layer of the NN via the one or more hardware processors, (i) a weight for each feature amongst the concatenated features set, and (ii) a bias for the concatenated features set, to obtain an optimal combination of features set;
predict the plurality of amino acid residues as one of an epitope or a non-epitope based on the optimal combination of features set to obtain at least one of a set of epitopes and a set of non-epitopes; and
prioritize the set of epitopes using a plurality of attention scores capturing a T-B association by using the protein language model, to obtain a set of immunogenic B-cell epitopes.

5. The system as claimed in claim 4, wherein the second set of features comprises at least a contact number, a B-factor, a protrusion index (PI), and a half sphere exposure (HSE).

6. The system as claimed in claim 4, wherein the learned hidden representation comprises at least one of a first hidden representation type, and a second hidden representation type.

7. One or more non-transitory machine-readable information storage mediums comprising one or more instructions which when executed by one or more hardware processors cause:

receiving an antigen protein sequence as an input from a user, wherein the antigen protein sequence comprises a plurality of amino acid residues;
extracting, by using a protein language model, a first set of features for each position of the plurality of amino acid residues comprised in the antigen protein sequence;
transforming, by using a first set of linear layers of a Neural Network (NN), the first set of features into a learned hidden representation;
extracting a second set of features for each position of the plurality of amino acid residues comprised in the antigen protein sequence;
concatenating the second set of features and the learned hidden representations to obtain a concatenated feature set;
learning, by using a second set of linear layer of the NN, (i) a weight for each feature amongst the concatenated features set, and (ii) a bias for the concatenated features set, to obtain an optimal combination of features set;
predicting the plurality of amino acid residues as one of an epitope or a non-epitope based on the optimal combination of features set to obtain at least one of a set of epitopes and a set of non-epitopes; and
prioritizing the set of epitopes using a plurality of attention scores capturing a T-B association by using the protein language model, to obtain a set of immunogenic B-cell epitopes.

8. The one or more non-transitory machine readable information storage mediums as claimed in claim 7, wherein the

second set of features comprises at least a contact number, a B-factor, a protrusion index (PI), and a half sphere exposure (HSE).

**9.** The one or more non-transitory machine readable information storage mediums as claimed in claim 7, wherein the learned hidden representation comprises at least one of a first hidden representation type, and a second hidden representation type.

SYSTEM
100
MEMORY
102
DATABASE
108
HARDWARE PROCESSOR(S)
104
INTERFACE(S)
106

FIG. 1

ESM2 Embeddings
Encoder
Encoder
Encoder
……...
Encoder
Dense Network
X = Transformed feature
Concatenation
Linear layer $W_1X$ + $W_2S'$ + bias
CEp/Not CEp
Antigen sequence … D I V E Q M …
Structural Features – Contact number, B-Factor, Protrusion index S'

FIG. 2

ESM2 Embeddings
Encoder
Encoder
Encoder
Encoder
Layer 1
Layer 2
Layer 3
Layer 4
X
transformed vector X
Linear layer
$W_1X + W_2S'$
+ bias
CEp or Non-CEp
1280D vector
Antigen sequence
… D I V **E** Q M ...
Structural features


FIG. 3

receiving, via one or more hardware processors, an antigen protein sequence as an input from a user, wherein the antigen protein sequence comprises a plurality of amino acid residues
202
extracting, by using a protein language model via the one or more hardware processors, a first set of features for each position of the plurality of amino acid residues comprised in the antigen protein sequence
204
transforming, by using a first set of linear layers of a Neural Network (NN) via the one or more hardware processors, the first set of features into a learned hidden representation
206
extracting, via the one or more hardware processors, a second set of features for each position of the plurality of amino acid residues comprised in the antigen protein sequence
208
concatenating, via the one or more hardware processors, the second set of features and the learned hidden representations to obtain a concatenated feature set
210
learning by using a second set of linear layer of the NN via the one or more hardware processors, (i) a weight for each feature amongst the concatenated features set, and (ii) a bias for the concatenated features set, to obtain an optimal combination of features set
212
predicting, via the one or more hardware processors, the plurality of amino acid residues as one of an epitope or a non-epitope based on the optimal combination of features set to obtain at least one of a set of epitopes and a set of non-epitopes
214
prioritizing, via the one or more hardware processors, the set of epitopes using a plurality of attention scores capturing a T-B association by using the protein language model, to obtain a set of immunogenic B-cell epitopes
216

**FIG. 4**

Receiver Operating Characteristic Curve
True Positive Rate
False Positive Rate
0.0
0.2
0.4
0.6
0.8
1.0
Discotop2.0 (AUC = 0.619)
Discotop3.0 (AUC = 0.755)
Bepipred2.0 (AUC = 0.560)
Bepipred3.0 (AUC = 0.660)
MLP_BF+PI_II _4_layer (AUC = 0.829)
MLP_BF_II _4_layer (AUC = 0.817)
MLP_BF+CN+PI_II _4_layer (AUC = 0.826)
MLP_CN_I _4_layer (AUC = 0.796)
MLP_HSE_I _4_layer (AUC = 0.793)
Random (.5)

**FIG. 5**

Receiver Operating Characteristic Curve
True Positive Rate
False Positive Rate
0.0
0.2
0.4
0.6
0.8
1.0
Bepipred2.0 (AUC = 0.47)
Bepipred3.0 (AUC = 0.50)
Epidope (AUC = 0.56)
CEP_BF_II_4_layer (AUC = 0.67)
LEP_BF_II_4_layer (AUC = 0.75)
LEP_BF+PI_II_4_layer (AUC = 0.77)
LEP_CN+BF+PI_II_4_layer (AUC = 0.78)
Random (.5)

FIG. 6

Percentage of B-cell Epitopes attended
0 B-cell epitopes attended
100% B-cell epitopes attended
Density
Prediction scores
0.00
0.25
0.50
0.75
1.00
1.25
1.50
1.75
2.00
0.0
0.2
0.4
0.6
0.8
1.0

FIG. 7

Percentage of B-cell Epitopes attended
0 B-cell epitopes attended
100% B-cell epitopes attended
Density
Prediction scores
0.0
0.5
1.0
1.5
2.0
2.5
0.0
0.2
0.4
0.6
0.8
1.0

FIG. 8

Europäisches
Patentamt
European
Patent Office
Office européen
des brevets

# EUROPEAN SEARCH REPORT

Application Number
EP 26 16 3095

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZENG YUANSONG ET AL: "Identifying B-cell epitopes using AlphaFold2 predicted structures and pretrained language model", BIOINFORMATICS (ONLINE), vol. 39, no. 4, 3 April 2023 (2023-04-03), XP093131201, GB, US, NL ISSN: 1367-4811, DOI: 10.1093/bioinformatics/btad187 Retrieved from the Internet: URL:https://academic.oup.com/bioinformatics/article-pdf/39/4/btad187/50087105/btad187.pdf> * pp. 2-3; figure 1 * | 1-9 | INV. G16B20/30 G16B40/20 |
| A | ZHU JAMES ET AL: "BepiTBR: T-B reciprocity enhances B cell epitope prediction", ISCIENCE, vol. 25, no. 2, 12 January 2022 (2022-01-12), pages 1-19, XP093415263, DOI: 10.1016/j.isci Retrieved from the Internet: URL:https://www.sciencedirect.com/science/article/pii/S2589004222000347?via%3Dihub> * p. 1 * | 1-9 | |
| | -/-- | | TECHNICAL FIELDS SEARCHED (IPC) |
| | | | G16B |

The present search report has been drawn up for all claims

1

| Place of search | Date of completion of the search | Examiner |
|---|---|---|
| Munich | 17 July 2026 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt
European
Patent Office
Office européen
des brevets

# EUROPEAN SEARCH REPORT

Application Number
EP 26 16 3095

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | Sajeed Rakshanda ET AL: "An improved deep learning model for immunogenic B epitope prediction", , 1 August 2025 (2025-08-01), pages 1-22, XP093415249, DOI: 10.1101/2025.07.29.667398 Retrieved from the Internet: URL:https://www.biorxiv.org/content/10.1101/2025.07.29.667398v1.full * the whole document * ----- | 1-9 | |

TECHNICAL FIELDS SEARCHED (IPC)

The present search report has been drawn up for all claims

1

| Place of search | Date of completion of the search | Examiner |
|---|---|---|
| Munich | 17 July 2026 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- IN 202521029943


### Non-patent literature cited in the description


- **BAHAI et al.** EpitopeVec: linear epitope prediction using deep protein sequence embeddings. *Bioinformatics*, 2021, vol. 37, 4517-4525
- **SANCHEZ-TRINCADO et al.** Fundamentals and Methods for T- and B-Cell Epitope Prediction. *Journal of Immunology Research*, 2017, 2680160
- **CLIFFORD et al.** Improved B - cell epitope prediction using protein language models. *Protein Science: A Publication of the Protein Society*, 2022, vol. 31, e4497
- **JUMPER et al.** Highly accurate protein structure prediction with AlphaFold. *Nature*, 2021, vol. 596, 583-589
- **IVANISENKO et al.** SEMA 2.0: web-platform for B-cell conformational epitopes prediction using artificial intelligence. *Nucleic Acids Research*, 2024, vol. 52, W533-W539
- **SU et al.** Protein language modeling with structure-aware vocabulary. *bioRxiv*, 2023, 10
- **JESPERSEN et al.** BepiPred-2.0: improving sequence-based B-cell epitope prediction using conformational epitopes. *Nucleic Acids Research*, 2017, vol. 45, W24
- **PONOMARENKO et al.** ElliPro: a new structure-based tool for the prediction of antibody epitopes. *BMC Bioinformatics*, 2008, vol. 9, 514
- **KRINGELUM et al.** Reliable B Cell Epitope Predictions: Impacts of Method Development and Improved Benchmarking. *PLoS Computational Biology*, 2012, vol. 8, e1002829
- **ZHOU et al.** SEPPA 3.0-enhanced spatial epitope prediction enabling glycoprotein antigens. *Nucleic Acids Research*, 2019, vol. 47, W388
- **SILVA et al.** epitope3D: a machine learning method for conformational B-cell epitope prediction. *Brief Bioinform*, 2022, vol. 23, bbab423
- **REN et al.** Tertiary structure-based prediction of conformational B-cell epitopes through B factors. *Bioinformatics*, 2014, vol. 30, 1264-273
- **XIA et al.** APIS: accurate prediction of hot spots in protein interfaces by combining protrusion index with solvent accessibility. *BMC Bioinformatics*, 2010, vol. 11, 174
- **SWEREDOSKI** ; **BALDI**. PEPITO: improved discontinuous B-cell epitope prediction using multiple distance thresholds and half sphere exposure. *Bioinformatics*, 2008, vol. 24, 1459-1460
- **BURLEY et al.** The Single Global Macromolecular Structure Archive. *Methods Mol Biol*, 2017, vol. 1607, 627-641
- **HOU et al.** SeRenDIP-CE: sequence-based interface prediction for conformational epitopes. *Bioinformatics*, 2021, vol. 37, 3421-3427
- **DESTA et al.** The ClusPro AbEMap web server for the prediction of antibody epitopes. *Nature protocols*, 2023, vol. 18, 1814
- **KRAWCZYK et al.** Improving B-cell epitope prediction and its application to global antibody-antigen docking. *Bioinformatics*, 2014, vol. 30, 2288
- **BARLOW et al.** Continuous and discontinuous protein antigenic determinants. *Nature*, 1986, vol. 322, 747-748
- **LANZAVECCHIA**. Antigen-specific interaction between T and B cells. *Nature*, 1985, vol. 314, 537-539
- **BIAVASCO** ; **DE GIOVANNI**. The Relative Positioning of B and T Cell Epitopes Drives Immunodominance. *Vaccines*, 2022, vol. 10, 1227